# EUROPÄISCHE PATENTANMELDUNG

(11) **EP 0 950 705 A1**
(43) Veröffentlichungstag der Anmeldung: **20.10.1999**
(21) Anmeldenummer: 99106864.4
(22) Anmeldetag: 07.04.1999
(51) Int. Cl.: C11D 17/00, C11D 17/04, A61K 7/50

(54) **Verwendung von Pflege- und Reinigungszubereitungen zur Befeuchtung von Reinigungstüchern**

(30) Priorität: 18.04.1998 DE 19817301
(71) Anmelder: Bode Chemie GmbH & Co., D-22525 Hamburg (DE)
(72) Erfinder: Westphal,Ursula, 22299 Hamburg (DE)
(74) Vertreter: Dinné, Erlend

(57) **Zusammenfassung**

Verwendung von O/W-Mikroemulsionen zur Befeuchtung von Reinigungstüchern aus Papier.

## Beschreibung

Die vorliegende Erfindung betrifft die Verwendung von Pflege- und Reinigungszubereitungen zur Befeuchtung von Reinigungstüchern, insbesondere zur Befeuchtung von Hygiene-Papiertüchern, wie beispielsweise Toilettenpapier oder Küchenpapier. Ferner betrifft die Erfindung die Verwendung von Pflege- und Reinigungszubereitungen zum Befeuchten von Papier durch Sprühanlagen.

Reinigungtücher aus Papier werden im Haushalt, aber auch im gewerblichen Bereich in großer Zahl eingesetzt. Sie werden beispielsweise zur Reinigung des Körpers angewendet, z. B. als Toilettenpapier, Taschentücher und Servietten, in der Babypflege, zum Abschminken oder zum Reinigen oder Trocknen der Hände, aber auch generell zum Säubern von Oberflächen und Geräten, z. B. in der Küche oder im Laborbetrieb. Meist werden die Reinigungtücher als trockene Papiere verwendet, in jüngster Zeit finden aber auch immer häufiger feuchte Papiere Verwendung. Die Befeuchtung soll dabei in der Regel dazu dienen, eine bessere Reinigung und darüber hinaus auch eine Pflege der zu reinigenden Flächen zu erzielen.

Um ein Austrocknen feuchter Tücher zu verhindern, müssen sie in einer feuchtigkeitsdichten Umhüllung verpackt werden. Der Stand der Technik kennt einzeln oder stapelweise verpackte Tücher.

Einzeln verpackte Tücher sind beispielsweise die sogenannten 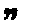 Erfrischungstücher", welche in Flugzeugen oder Restaurants zum Reinigen der Hände ausgegeben werden. Die Verwendung dieser Tücher in Bereichen mit hohem Verbrauch empfiehlt sich wegen der notwendigen Entfernung der aufwendigen Verpackung von jedem einzelnen Blatt allerdings nicht. Zudem sind diese Tücher aufgrund ihrer Verpackung sowohl in der Herstellung als auch in der Anwendung relativ teuer, und bei ihrer Verwendung fallen erhebliche Mengen von Abfall an.

Stapelweise verpackte Tücher sind zwar, wenn die feuchtigkeitsdichte Verpackung einmal geöffnet wurde, leichter und schneller aus dieser entnehmbar als einzeln verpackte, allerdings trocknen sie leicht aus, wodurch etwaige Wirkstoffe ihre Wirksamkeit verlieren können, insbesondere, wenn die Verpackung nicht ausreichend dicht wiederverschließbar ist. Zudem kann bei einer öffentlichen Benutzung die notwendige Hygiene nicht gewährleistet werden, da leicht Fremdkörper in die Packung eindringen können.

Für viele praktische Anwendungsfälle ist es zudem vorteilhaft, wenn sowohl trockene als auch feuchte Reinigungstücher vorhanden sind, welche ohne großen Aufwand unmittelbar nacheinander verwendet werden können.

Eine relativ neue technische Entwicklung sind Spender für Reinigungstücher, aus welchen diese, je nach Bedarf, entweder trocken oder feucht entnommen werden können. Die Deutsche Offenlegungsschrift 196 30 686 beschreibt einen Spender für Reinigungstücher, wobei das auf Rollen vorgehaltene Papier mittels einer Sprühdüse befeuchtet werden kann.

Die Anforderungen an die in einem derartigen Befeuchtungsspender zu verwendende Befeuchtungsflüssigkeit sind im folgenden aufgelistet:
- Sie soll die Reinigung fördern,
- sie soll pflegend wirken,
- sie soll sowohl galenisch als auch mikrobiologisch stabil sein,
- sie muß versprühbar sein und darf sich beim Sprühen nicht entmischen und
- das mit ihr befeuchtete Papier darf beim Entnehmen nicht zerreißen.

Dem Stand der Technik mangelt es an Zubereitungen, welche sich zum Befeuchten von Papier in automatischen Spendern eignen. Befeuchtet man beispielsweise Hygiene-Papier mit einfachem Wasser oder wäßrigen Lösungen, so zerreißt das Papier leicht bei der Förderung in der Maschine bzw. bei der Entnahme aus dem Spender. Eine Befeuchtung mit Öl hingegen hat den Nachteil, daß das Papier unregelmäßig befeuchtet wird, da sich Öl nicht fein verteilt sprühen läßt. Übliche Emulsionen wiederum haben den Nachteil, daß sie beim Versprühen zerstört werden, was zu einer unregelmäßigen Befeuchtung des Papiers und insbesondere auch Wirkstoffverteilung führt.

ln üblichen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca. 1 µm bis ca. 50 µm. Solche 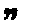 Makroemulsionen" sind, ohne weitere färbende Zusätze, milchigweißgefärbt und opak. Feinere 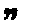 Makroemulsionen", deren Tröpfchendurchmesser im Bereich von ca. 10⁻¹ µm bis ca. 1 µm liegen, sind, wiederum ohne färbende Zusätze, bläulichweißgefärbt und undurchsichtig. In Mikroemulsionen hingegen liegt der Tröpfchendurchmesser im Bereich von etwa 10⁻² µm bis etwa 10⁻¹ µm. Sie werden mit abnehmender Tröpfchengröße zunehmend klarer und transparenter. Mikroemulsionen sind meist niedrigviskos. Die Viskosität vieler Mikroemulsionen vom O/W-Typ ist vergleichbar mit der des Wassers.

Vorteil von Mikroemulsionen ist, daß in der dispersen Phase Wirkstoffe feiner verteilt vorliegen können als in der dispersen Phase von 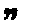 Makroemulsionen". Ein weiterer Vorteil ist, daß sie aufgrund ihrer niedrigen Viskosität ohne eine Phasentrennung versprühbar sind.

Aufgabe der vorliegenden Erfindung war es, die Nachteile des Standes der Technik zu beseitigen und Pflege- und Reinigungszubereitungen zu finden, welche sich zum Befeuchten von Reinigungstüchern, insbesondere zum Befeuchten von Papier durch Sprühanlagen eignen.

Es war indes überraschend und für den Fachmann nicht vorauszusehen, daß die Verwendung von O/W-Mikroemulsionen zur Befeuchtung von Reinigungstüchem den geschilderten Nachteilen des Standes der Technik Abhilfe schaffen würden.

Als Mikroemulsionen werden im Sinne der vorliegenden Erfindung Emulsionen bezeichnet, deren Tröpfchendurchmesser kleiner als 80 nm ist.

Die erfindungsgemäße Verwendung von O/W-Mikroemulsionen liefert in jeglicher Hinsicht überaus befriedigend befeuchtete Reinigungstücher. Es war insbesondere überraschend, daß die Reißfestigkeit von erfindungsgemäß befeuchteten Hygiene-Papieren nicht vermindert wird, so daß diese sich beispielsweise aus einem Befeuchtungsspender zerstörungsfrei entnehmen lassen. Mit erfindungsgemäß zu verwendenden O/W-Mikroemulsionen lassen sich alle üblichen Hygiene-Papiere, insbesondere alle gängigen, wie beispielsweise die nachstehend genannten, Toilettenpapiere befeuchten.

Als Toilettenpapiere werden heutzutage in der Regel sogenannte Krepp-Papiere oder Tissues verwendet. Krepp-Papiere sind durch Naß- oder Trocken-Kreppung dehnbar und schmiegsam gemachte Papiersorten; Tissue ist ein besonders dünnes, weiches, überweigend holzfreies Material mit feiner (Trocken-)Kreppung, welches aus einer oder mehreren Lagen besteht und sehr saugfähig ist. Das Flächen-Gewicht der Einzellage liegt im allgemeinen bei weniger 25 g/m² vor der Kreppung. Die Papiere können neben den Faserstoffen auch noch weitere Stoffe, sogenannte Papierhilfsmittel enthalten. Hierzu gehören Füllstoffe (z. B. Kaolin, Kreide, Titandioxid) zur Verbesserung von Glätte und Bedruckbarkeit sowie zur Oberflächenvergütung, Farbstoffe und Pigmente zur Einfärbung oder Oberflächenfärbung, Bindemittel (z. B. Stärke, Kasein und andere Proteine, Kunststoff-Dispersionen, Harzleime und dergleichen mehr) zur Verfestigung des Fasergefüges sowie zur Bindung von Füllstoffen und Pigmenten und zur Erhöhung der Wasserfestigkeit, optische Aufheller zur Erhöhung des Weißgrades, Retentions-Mittel (z. B. Aluminiumsulfat und synthetische kationische Stoffe) zur Rückhaltung der Fein- und Füllstoffe während der Herstellung, 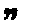 De-Inking-Chemikalien" zur Aufbereitung von Altpapier sowie diverse weitere Stoffe, wie z. B. Netzmittel, Entschäumer, Konservierungsmittel, Schleimbekämpfungsmittel, Weichmacher, Antiblockmittel, Antistatika, Hydrophobierungsmittel usw.

Die Gesamtmenge an Ölen in den erfindungsgemäß zu verwendenden Mikroemulsionen wird vorteilhaft gewählt aus dem Bereich 0,05 bis 20 Gew.-%, insbesondere 1 bis 15 Gew.-%, bezogen auf das Gesamtgewicht der Zubereitung. Die Ölphase kann aus einer oder mehreren Komponenten bestehen.

Die Komponenten der Ölphase werden vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner können die Ölkomponenten vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Auch beliebige Abmischungen solcher Öl- und Wachskomponenten sind vorteilhaft im Sinne der vorliegenden Erfindung einzusetzen.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Vorteilhaft wird die Ölkomponente oder werden die Ölkomponenten gewählt aus der Gruppe Di-n-Octylether, Capryl/Caprinsäureester, Capryl/Caprinsäuretriglycerid und Octyldodecanol. Besonders vorteilhaft erfindungsgemäß zu verwendende Mikroemulsionen enthalten 2 bis 6 Gew.-% Di-n-Octylether, 2 bis 6 Gew.-% Capryl/Caprinsäureester, 1 bis 6 Gew.-% Capryl/Caprinsäuretriglycerid und/oder 1 bis 6 Gew.-% Octyldodecanol.

Es ist besonders vorteilhaft im Sinne der vorliegenden Erfindung, O/W-Mikroemulsionen zu verwenden, welche nach dem Phasen-Inversions-Temperatur-Verfahren (PIT-Verfahren) erhältlich sind.

Hydrophile Emulgatoren, namentlich polyethoxylierte und polypropoxylierte Emulgatoren, ändern bei steigender Temperatur ihr Löslichkeitsverhalten von wasserlöslich zu fettlöslich. Der Temperaturbereich, in dem die Emulgatoren ihre Löslichkeit änderen, wird Phasen-Inversions-Temperaturbereich genannt.

Erfindungsgemäß vorteilhaft zu verwendende O/W-Mikroemulsionen können erhalten werden, wenn der Anteil des Emulgators oder der Emulgatoren unter 5 Gew.-%, insbesondere zwischen 3 und 5 Gew.-% liegt, bezogen auf das Gesamtgewicht der Zubereitung.

Für eine Mischung aus Wasser, Öl und Emulgatoren, welche unterhalb des Phasen-Inversions-Temperaturbereichs nach Rühren eine O/W-Emulsion ergibt, äußert sich die Änderung des Löslichkeitsverhaltens darin, daß sie, wenn sie auf eine Temperatur oberhalb des Phasen-Inversions-Temperaturbereichs gebracht wird, schließlich eine W/O-Emulsion ergibt. Wird diese Emulsion wieder abgekühlt, wird erneut eine O/W-Emulsion erhalten, welche aber eine kleinere Tröpfchengröße von bis zu 100 nm besitzt.

Die erfindungsgemäß zu verwendenden Mikroemulsionen sind nach bekannten Verfahren erhältlich. Sie können kosmetische oder dermatologische Hilfsstoffe enthalten, wie sie üblicherweise in solchen Zubereitungen verwendet werden, z. B. Pflegestoffe, wie Panthenol, Allantoin, Bisabolol und dergleichen mehr, zusätzliche Wirkstoffe wie Vitamine oder Proteine, Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Werden zusätzliche Pflegestoffe verwendet, so ist es vorteilhaft, ihren Gehalt zwischen 0,05 und 2 Gew.-% bezogen auf das Gesamtgewicht der Zubereitung zu wählen.

Die jeweils einzusetzenden Mengen an kosmetischen oder dermatologischen Trägerstoffen und Parfüm können in Abhängigkeit von der Art des jeweiligen Produktes vom Fachmann durch einfaches Ausprobieren leicht ermittelt werden.

Das nachfolgende Beispiel soll die vorliegende Erfindung verdeutlichen, ohne sie einzuschränken. Die Zahlenwerte bedeuten Gewichtsteile, bezogen auf das Gesamtgewicht der Zubereitung.

### Beispiel:

### Gewichtsteile

- 4,5: C₁₀₋₁₂-Alkyl-C₈₋₁₀-Fettsäureester
- 0,5: Allantoin
- 0,75: D-Panthanol
- 4,5: Di-n-Cetylether
- 3,0: Pentandiol
- 2,7: Glycerinmonostearat
- 0,36: Stearylalkoholpolyethylenoxid (12 EO) ether
- 0,32: Cetylstearylalkohol
- 0,23: Cetylpalmitat
- 1,9: Cetylalkoholpolyethylenoxid (20 EO) ether
- 0,2: Parfüm
- ad 100: Wasser

Die Bestandteile wurden der Reihe nach bei Raumtemperatur in Wasser gelöst und unter Rühren auf 85 °C erwärmt. Bei dieser Temperatur wurde 5 Minuten homogenisiert (Becomix). Anschließend wurde langsam (über 2 Stunden) unter Rühren auf 25 °C abgekühlt.

Diese Mikroemulsion wurde in einen Befeuchtungsspender gemäß der Deutschen Offenlegungsschrift 196 30 686 eingefüllt und auf ein dreilagiges Toilettenpapier gesprüht (0,2 bis 0,5 ml pro Blatt) Das befeuchtete Papier ließ sich ohn Zerreißen entnehmen und hinterließ ein angenehmes Hautgefühl.

## Patentansprüche

1. Verwendung von O/W-Mikroemulsionen zur Befeuchtung von Reinigungstüchem aus Papier.

2. Verwendung nach Anspruch 1, dadurch gekennzeichnet, daß die Größe der Öltröpfchen in der Mikroemulsion kleiner als 80 nm ist.

3. Verwendung nach Anspruch 1, dadurch gekennzeichnet, daß die Gesamtmenge an Ölen in der Mikroemulsion gewählt wird aus dem Bereich 0,05 bis 20 Gew.-%, insbesondere 1 bis 15 Gew.-%, bezogen auf das Gesamtgewicht der Zubereitung.

4. Verwendung nach Anspruch 1, dadurch gekennzeichnet, daß die Ölphase der Mikroemulsion Di-n-Octylether, Capryl/Caprinsäureester, Capryl/Caprinsäuretriglycerid oder Octyldodecanol oder deren Gemische enthält.

5. Verwendung nach Anspruch 1, dadurch gekennzeichnet, daß die Gesamtmenge an Emulgatoren in der Mikroemulsion unter 5 Gew.-%, insbesondere zwischen 3 und 5 Gew.-% liegt, bezogen auf das Gesamtgewicht der Zubereitung.

6. Verwendung nach Anspruch 1, dadurch gekennzeichnet, daß die Mikroemulsion nach dem Phasen-Inversions-Temperatur-Verfahren erhältlich ist.

7. Verwendung nach Anspruch 1, dadurch gekennzeichnet, daß das Reinigungstuch als Toilettenpapier verwendet wird.

8. Mit einer O/W-Mikroemulsionen befeuchtetes Toilettenpapier.
